# EUROPÄISCHE PATENTSCHRIFT

(11) **EP 1 474 195 B1**
(45) Veröffentlichungstag und Bekanntmachung des Hinweises auf die Patenterteilung: **07.12.2005**
(21) Anmeldenummer: 02710673.1
(22) Anmeldetag: 14.02.2002
(51) Int. Cl.: A61M 15/00

(54) **INHALATIONSHILFSGERÄT**
INHALATION AID
APPAREIL AUXILIAIRE D'INHALATION

(43) Veröffentlichungstag der Anmeldung: 10.11.2004
(73) Patentinhaber: atmed Aktiengesellschaft, 83435 Bad Reichenhall (DE)
(72) Erfinder: KLEIN, Christoph, A-5084 Grossgmain-Hinterreit (AT)
(74) Vertreter: Miksovsky, Alexander
(86) Internationale Anmeldenummer: PCT/AT2002/000051
(87) Internationale Veröffentlichungsnummer: WO 2003/068299

(56) Entgegenhaltungen:
- EP-A- 0 385 212
- WO-A-00/33902
- WO-A-01/87396
- DE-A- 19 941 236
- US-A- 5 809 996

## Beschreibung

Die vorliegende Erfindung bezieht sich auf ein Inhalationshilfsgerät zur Verwendung in Verbindung mit einem Aerosolabgabegerät, umfassend eine Kammer, welche mit einer Öffnung für die Verbindung mit einem Aerosolabgabegerät und mit einer Öffnung mit einem Mundstück zum Abziehen des Aerosols ausgebildet ist, wobei die Kammer mit veränderbarem Volumen ausgebildet ist.

Dosier-Aerosole sind überwiegend eingesetzte Verabreichungsformen von Medikamenten zur Behandlung von Asthma bronchiale und chronischer Bronchitis. Auch bei Allergien kommen Dosier-Aerosole immer mehr zur Anwendung. Die inhalative Behandlung dieser Krankheiten mit Dosier-Aerosolen hat sich hiebei besonders bei akut auftretenden Beschwerden, wie Atemnot, als sehr wirkungsvoll erwiesen. Ein Dosier-Aerosol ist in der Regel in einem Medikamentenbehälter enthalten, welcher die einzelnen Komponenten, nämlich den mikronisierten Wirkstoff, Suspendierungsmittel und Treibgas beinhaltet. Durch Schütteln vor der Anwendung werden die einzelnen Komponenten vermischt. Nach jedem Sprühstoß werden diese Komponenten, wenn der Medikamentenbehälter senkrecht gehalten wird, der Dosierkammer des Medikamentenbehälters in genormter Menge neuerlich zugeführt und nur die in der Dosierkammer enthaltene Menge kann bei einem Sprühstoß ausgebracht werden.

Um die treibgashaltigen Dosier-Aerosole inhalieren zu können, werden Aerosolabgabegeräte, welche überwiegend L-förmig ausgebildet sind, wie dies beispielsweise der US-PS 3 994 421, der US-PS 4 509 515, der DE-OS 32 29 702 oder der DE-OS 38 16 276 zu entnehmen ist, eingesetzt. Die L-förmigen Aerosolabgabegeräte haben den großen Nachteil, daß bei diesen das Aerosol unkontrolliert und mit sehr hoher Geschwindigkeit aus dem Mundstück des Abgabegerätes austritt und so ein großer Teil des Aerosols, meist mehr als 80 %, bei jedem Sprühstoß im Mund-, Rachen- und Gaumenbereich kleben bleibt. Nur ca. 6 bis 20 % des Aerosols gelangen in den Respirationstrakt, wo sie ihren therapeutischen Effekt entwickeln können. Der hohe, im Mund-, Rachen- und Gaumenbereich verbleibende Medikamentenanteil kann hiebei bei diversen Dosier-Aerosolen unangenehme Nebenwirkungen verursachen, die gegebenenfalls wiederum mit zusätzlichen Medikamenten behandelt werden müssen. Bei derartigen Aerosolabgabegeräten dient somit der Mund-, Rachen- und Gaumenbereich als Zerstäubungskammer.

Alle Dosier-Aerosole sind im Funktionsaufbau im wesentlichen gleich. Die üblicherweise in dem Medikamentenbehälter enthaltene Suspension enthält mikronisierte Wirkstoffpartikel. Bei den üblichen L-förmigen Aerosolabgabegeräte sind die mikronisierten Wirkstoffpartikel beim Austritt aus der Düse mit sogenannten Primärtröpfchen ummantelt. Die Primärtröpfchen sind beim Düsenaustritt normalerweise so groß, daß sie nicht schwebfähig sind und wirken daher in dem Mund-, Rachen- und Gaumenbereich wie Geschosse. Darum werden aufbauend auf den L-förmigen Aerosolabgabegeräten weitere spezielle Inhalationshilfsgeräte, sogenannte Spacer, angeboten, die zu einer Verminderung der Medikamentenablagerungen im Mund-, Rachen- und Gaumenbereich führen sollen, wie dies beispielsweise aus der EP-A 0 009 667 bekannt geworden ist. Eine weitere Ausbildung eines Inhalationshilfsgerätes ist auch der EP-A 0 384 050 zu entnehmen, wobei ein in seinem Volumen nicht veränderbarer Zylinder an seinem Eintrittsende mit veränderbarem Querschnitt und an seinem Austrittsende mit einem Ventil ausgebildet ist.

Diese Inhalationshilfsgeräte sind großvolumige Zerstäubungskammern, in welche das Aerosol zuerst mit den L-förmigen Aerosolabgabegeräten hineingesprüht werden soll. Die beim Sprühstoß gebildeten Primärtröpfchen haben somit eine ausreichende Strecke zur Verfügung, um ihre hohe Geschwindigkeit abzubremsen. Durch das große Volumen des Spacers werden die Primärtröpfchen darüber hinaus angewärmt und können somit verdampfen. Nach dem Verdampfen sollen hiebei möglichst viele Wirkstoffpartikel, die kleiner als 5 µm und somit lungengängig sind, im Spacer schweben, wobei alle nicht schwebfähigen Medikamentenpartikel sich an der Wand des Spacers anlagern und dort verbleiben.

Wird bei der Anwendung von Dosier-Aerosolen mit üblichen L-förmigen Aerosolabgabegeräten kein derartiges zusätzliches Inhalationshilfsgerät verwendet, so schießen die Primärtröpfchen aufgrund ihrer Größe wie Geschosse in den Mundraum und bleiben, wie oben beschrieben, zum größten Teil an der Rachenwand kleben, weil keine ausreichende Strecke und kein ausreichendes Volumen vorhanden ist, damit die Primärtröpfchen verdampfen und ihre hohe Geschwindigkeit reduzieren können.

Bei der Anwendung eines Spacers bzw. Inhalationshilfsgeräts in Kombination mit einem Aerosolabgabegerät verlagert sich dagegen die bisherige Mund-, Rachen- und Gaumendeposition des Dosier-Aerosols in den Spacer und somit kann die Gefahr von Nebenwirkungen, wie Mundsoor oder Entzündungen, nahezu gebannt bzw. weitestgehend verringert werden. Die Medikamentendeposition in der Lunge erhöht sich nur minimal. Ein weiterer Vorteil des Spacers besteht darin, daß aufgrund des großen Volumens die schwebfähigen Wirkstoffpartikel einige Zeit im Spacer verbleiben und der Anwender genügend Zeit hat, das Aerosol aus dem Spacer zu inhalieren. Somit konnte weiters das Koordinationsproblem eines gleichzeitigen Auslösens eines Sprühstoßes des Aerosols und des Inhalierens gelöst werden. Der Spacer ist ein sinnvolles Produkt, um die Therapie mit den Dosier-Aerosolen zu verbessern, die subjektiven Nebenwirkungen zu verringern und das Koordinationsproblem zu lösen.

Das größte Problem bei der Nutzung eines derartigen Inhalationshilfsgerätes bzw. Spacers besteht allerdings in seinen großdimensionierten Abmessungen und derartige Spacer sind daher aufgrund ihrer Größe als Taschengeräte ungeeignet und deshalb auch unpraktisch zu verwenden. Somit wird es für den Anwender fast unmöglich, den Spacer immer mit sich zu führen. Die Anwender von Dosier-Aerosolen müssen in der Regel mehrmals täglich ihre Dosier-Aerosole benutzen und würden hiebei gerne auf den Spacer als sinnvolles Hilfsmittel zurückgreifen.

Probleme mit großvolumigen Inhalationshilfsgeräten werden teilweise dadurch gemindert, daß derartige Spacer mit veränderbarem Volumen vorgeschlagen werden, wie dies beispielsweise der GB-A 2 110 543, der GB-A 2 182 249, der US-A 5 074 294, der WO 97/12638, der US-A 5 571 246, der GB-A 2 301 040 oder der WO 96/37249 zu entnehmen ist. Bei diesen bekannten Inhalationshilfsgräten ist jedoch nachteilig, daß sie sehr komplizierte technische Konstruktionen darstellen, welche einen übermäßig großen konstruktiven Aufwand erfordern und somit nicht kostengünstig herstellbar sind.

Aus der WO 97/12638, der US-A 5 571 246 und der GB-A 2 301 040 sind hiebei Ausführungsformen bekannt geworden, bei welchen zur Erzielung einer pyramidenartigen bzw. trichterartigen Form des Spacers bzw. Inhalationshilfsgeräts eine Mehrzahl von ineinander eingreifenden bzw. in zusammengeschobenem Zustand ineinander liegenden, sich konisch verjüngenden Ringen vorgesehen ist, welche relativ zueinander in Richtung der Längsachse des Inhalationshilfsgeräts verschiebbar sind. Nachteilig bei dieser bekannten Konstruktion ist die Tatsache, daß eine ausreichende Dichtheit zwischen den einzelnen Ringen nicht ohne weiteres erzielbar ist, so daß einerseits ein Verlust des Wirkstoffs bzw. Medikaments befürchtet werden muß und andererseits ein Eindringen von gegebenenfalls schädlichen Teilchen aus der Außenumgebung während des Aufnahmevorgangs des Medikaments nicht ausgeschlossen werden kann. DE 19 941 236 offenbart eine Inhalationshilfe mit der Merkmale im Oberbegriff des Anspruchs 1.

Ein weiteres Problem bei bekannten Inhalationshilfsgeräten besteht darin, daß jeder Anbieter von Dosier-Aerosolen üblicherweise seinen eigenen Spacer anbietet, der nur mit den an diesen angepaßten, eigenen Aerosolabgabegeräten und Dosier-Aerosolen verwendbar ist. Damit wird es dem Anwender nahezu unmöglich gemacht Produkte von einem anderen Hersteller in Verbindung mit dem vorhandenen Spacer zu verwenden.

Die vorliegende Erfindung zielt nun darauf ab, ausgehend von einem Inhalationshilfsgerät bzw. Spacer der eingangs genannten Art, dieses bzw. diesen so weiterzubilden, daß bei einfacher Konstruktion eine zuverlässige und insbesondere dichte Konstruktion des Inhalationshilfsgeräts zur Verfügung gestellt wird, welches in eine Transportposition mit verringertem Platzbedarf und eine Einsatzposition, bei welcher ein entsprechend großes Volumen der Kammer des Inhalationshilfsgeräts bereitgestellt wird, bringbar ist.

Zur Lösung dieser Aufgabe ist das erfindungsgemäße Inhalationshilfsgerät ausgehend von einem Inhalationshilfsgerät bzw. Spacer der eingangs genannten Art im wesentlichen dadurch gekennzeichnet, daß der Faltenbalg wenigstens eine ringartige Verstärkung mit gegenüber den anschließenden Bereichen des Faltenbalgs verstärktem Materialquerschnitt aufweist. Durch eine derartige ringartige Verstärkung bzw. eine Mehrzahl von ringartigen Verstärkungen mit entsprechend voneinander verschiedenem Durchmesser bzw. lichtem Querschnitt läßt sich zum einen eine entsprechende Verstärkung des Faltenbalgs erzielen, welche eine zuverlässige Einnahme einer Gebrauchsposition unter Aufrechterhaltung des gewünschten Kammervolumens sicherstellt. Darüber hinaus läßt sich durch wenigstens eine derartige ringartige Verstärkung bzw. eine Mehrzahl von aufeinander abgestimmten, ringartigen Verstärkungen ein gezieltes Falten bzw. Zusammenlegen in die einen kleineren Platzbedarf aufweisende Transportposition erzielen, wobei durch Vorsehen von mehreren ringartigen Verstärkungen mit entsprechend der konischen Verjüngung des Faltenbalgs verringertem Durchmesser bzw. lichtem Querschnitt diese ringartigen Verstärkungen in zusammengefaltetem Zustand des Inhalationshilfsgeräts jeweils ineinander eintauchen bzw. voneinander aufgenommen werden.

Die Kammer ist von einem sich konisch verjüngenden Faltenbalg aus einem durchgehenden, elastischen Material begrenzt. Dadurch, daß die Kammer des Inhalationshilfsgeräts bzw. Spacers von einem sich konisch verjüngenden Faltenbalg aus einem durchgehenden, elastischen Material begrenzt ist, sind jegliche Dichtheitsprobleme, wie sie bei dem oben erläuterten Stand der Technik zu befürchten sind, ausgeschlossen. Weiters läßt sich ein derartiges elastisches Material aus einem gegenüber den zu verwendenden Medikamenten bzw. Stoffen resistenten Material bereitstellen. Darüber hinaus ist es möglich, die geforderten hygienischen Bedingungen mit einem derartigen durchgehenden, elastischen Material aufrecht zu erhalten. Weiters läßt sich ein derartiger Faltenbalg in einem einzigen Herstellungsvorgang in einer Form bzw. einem Werkzeug unter Einsatz eines entsprechenden, einheitlichen Materials herstellen. In diesem Zusammenhang wird gemäß einer bevorzugten Ausführungsform vorgeschlagen, daß der Faltenbalg aus einem Silikon hergestellt ist. Eine Verwendung von Silikon zur Herstellung des Faltenbalgs ermöglicht nicht nur eine einfache und kostengünstige Herstellung, sondern erlaubt auch die Einhaltung der für den Einsatzzweck erforderlichen, insbesondere hygienischen bzw. medizin-technischen Bedingungen.

Zur Erzielung der gewünschten, vereinfachten Handhabbarkeit sowohl bei der Einnahme der Gebrauchsstellung und der Definition des erforderlichen Kammervolumens als auch für ein Überführen in die einen kleinen Raumbedarf aufweisende Transportposition ist hiebei die Ausbildung bevorzugt so getroffen, daß die Außenfläche des Faltenbalgs im Bereich der zum sich verjüngenden Ende weisenden Stirnfläche (n) der ringartigen Verstärkung(en) stufenartig bzw. abgesetzt ausgebildet ist.

Zur Unterstützung des Faltvorgangs ist gemäß einer weiters bevorzugten Ausführungsform vorgesehen, daß der Faltenbalg im an die Verstärkungen(en) unmittelbar anschließenden Bereich, insbesondere an der zum Inneren gewandten Seitenfläche der Kammer, mit verringertem Materialquerschnitt ausgebildet ist. Durch derartige Bereiche mit verringertem Materialquerschnitt läßt sich insbesondere beim Überführen in die Transportposition durch die durch den verringerten Materialquerschnitt definierten Biegelinien ein einfaches Zusammenfalten des die Kammer definierenden Faltenbalgs erzielen.

Für eine geschützte Aufnahme des die Kammer definierenden Faltenbalgs in der Transportposition kann vorgesehen sein, daß der Faltenbalg in zusammengefaltetem Zustand in einem topfartigen, starren Gehäuse aufnehmbar ist, welches das Mundstück zum Abziehen des Aerosols aus der Kammer aufweist, wie dies einer weiters bevorzugten Ausführungsform des erfindungsgemäßen Inhalationshilfsgeräts entspricht. Ein derartiges topfartiges Gehäuse kann den Faltenbalg in zusammengefaltetem Zustand bzw. in der Transportposition weitestgehend aufnehmen bzw. überdecken, so daß eine Beschädigung des Faltenbalgs während des Transports vermieden werden kann.

Für eine ordnungsgemäße Festlegung des Faltenbalgs an dem das Mundstück aufweisenden Gehäuse ist gemäß einer weiters bevorzugten Ausführungsform vorgesehen, daß der Faltenbalg an seinem einen größeren Querschnitt aufweisenden Ende mit einem Wulst bzw. Flansch versehen ist, an welchem ein Haltering festlegbar ist, wobei in diesem Zusammenhang weiters bevorzugt ist, daß der Haltering mit dem Gehäuse zur Aufnahme des Faltenbalgs insbesondere lösbar verbindbar ist.

Durch eine derartige Kombination eines Halterings, welcher am Faltenbalg festlegbar ist, mit dem Gehäuse läßt sich nicht nur eine sichere Aufnahme des die Kammer begrenzenden Faltenbalgs am Gehäuse erzielen, sondern es ist durch die lösbare Festlegung auch sichergestellt, daß entsprechend den Anforderungen der Faltenbalg bzw. das Innere der Kammer regelmäßig und einfach gereinigt werden kann. Für ein einfaches und zuverlässiges Festlegen wird hiebei vorgeschlagen, daß die Verbindung zwischen dem Haltering und dem Gehäuse von einem Schraubverschluß oder einem Bajonettverschluß gebildet ist, wie dies einer weiters bevorzugten Ausführungsform des erfindungsgemäßen Inhalationshilfsgeräts entspricht.

Für eine ordnungsgemäße Festlegung der Einzelelemente des erfindungsgemäßen Inhalationshilfsgeräts aneinander ist darüber hinaus vorgesehen, daß am Haltering und/oder dem Gehäuse Markierungen bzw. Rast- oder Justierelemente vorgesehen sind. Derartige Markierungen bzw. Rast- oder Justierelemente erlauben eine zuverlässige und dichte Festlegung der Einzelteile aneinander sowie nach einer erfolgten Trennung der Einzelteile, beispielsweise für eine Reinigung einen vereinfachten und zuverlässigen Zusammenbau.

Zur Erzielung einer weiteren Schutzfunktion, insbesondere der Öffnung des Mundstücks sowie der für eine Verbindung mit einem Aerosol-Abgabegerät vorgesehenen, weiteren Öffnung wird darüber hinaus vorgeschlagen, daß Abdeck- bzw. Verschlußeinrichtungen für die Öffnungen der Kammer vorgesehen sind, wie dies einer weiters bevorzugten Ausführungsform des erfindungsgemäßen Inhalationshilfsgeräts entspricht.

Die Erfindung wird nachfolgend anhand von in der beiliegenden Zeichnung schematisch dargestellten Ausführungsbeispielen näher erläutert. In dieser zeigen:
Fig. 1 eine perspektivische Darstellung des erfindungsgemäßen Inhalationshilfsgeräts in einer ausgefahrenen Betriebsstellung;
Fig. 2 in einer zu Fig. 1 ähnlichen Darstellung das erfindungsgemäße Inhalationshilfsgerät in eingefahrener Position bzw. Transportposition;
Fig. 3 in perspektivischer Explosionsdarstellung den Faltenbalg des erfindungsgemäßen Inhalationshilfsgeräts mit einem teilweise gelösten Haltering;
Fig. 4 in einer zu Fig. 3 ähnlichen Explosionsdarstellung das erfindungsgemäße Inhalationshilfsgerät;
Fig. 5 eine schematische Seitenansicht des erfindungsgemäßen Inhalationshilfsgeräts in der Betriebsstellung; und
Fig. 6 eine Schnittansicht entlang der Linie VI-VI der Fig. 5 durch das erfindungsgemäße Inhalationshilfsgerät.

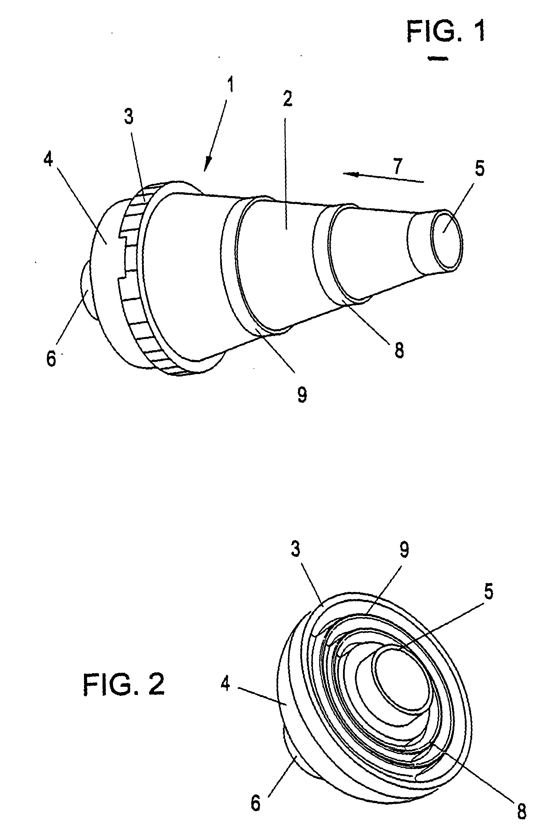

In den Figuren umfaßt das Inhalationshilfsgerät 1 jeweils einen eine Kammer definierenden, sich konisch verjüngenden Faltenbalg 2, an welchem, wie dies weiter unter noch im Detail erläutert werden wird, ein Haltering 3 festlegbar ist, wobei der Haltering 3, wie dies ebenfalls weiter unten im Detail erläutert werden wird, lösbar an einem topfartigen, starren Gehäuse 4 festlegbar ist. Wie dies aus einem Vergleich der Fig. 1 und 2 deutlich ersichtlich ist, definiert der sich konisch verjüngende Faltenbalg 2 aus einem durchgehenden, elastischen Material eine Kammer mit veränderbarem Volumen, wobei der Faltenbalg 2 an seinem einen geringeren Querschnitt aufweisenden Ende mit einer Öffnung 5 für eine Festlegung an einem nicht näher dargestellten Aerosol-Abgabegerät ausgebildet ist. Darüber hinaus ist das Inhalationshilfsgerät 1 an dem Gehäuse 4 mit einer ein Mundstück definierenden bzw. aufweisenden Öffnung 6 ausgebildet.

Alternativ zu der dargestellten, getrennten Ausführungsform des Faltenbalgs von dem Haltering 3 und dem Gehäuse 4 könnte auch an dem einen größeren Querschnitt aufweisenden Ende eine entsprechende einstückige Ausführungsform vorgesehen sein, welche wiederum eine das Mundstück definierende Öffnung 6 aufweist.

Aus der in Fig. 1 dargestellten Gebrauchs- bzw. Einsatzposition läßt sich der Faltenbalg 2 aus einem durchgehenden, elastischen Material entsprechend dem Pfeil 7 in die in Fig. 2 dargestellte, zusammengefaltete Position bzw. Transportstellung bringen, in welcher das Inhalationshilfsgerät 1 einen entsprechend verringerten Raum- bzw. Platzbedarf aufweist.

Der Faltenbalg 2 ist beispielsweise aus einem Silikon ausgebildet, wobei bei der dargestellten Ausführungsform in Abstand voneinander entlang der Erstreckung des sich konisch verjüngenden Faltenbalgs 2 zwei ringartige Verstärkungen 8 und 9 vorgesehen sind, welche einen unterschiedlichen Durchmesser bzw. eine unterschiedliche lichte Weite entsprechend der konischen Verjüngung des Faltenbalgs 2 in dem in Fig. 1 dargestellten, ausgefahrenen Zustand aufweisen.

Wie aus Fig. 2 ersichtlich, gelangen diese ringartigen Verstärkungen 8 bzw. 9 in der zusammengefalteten Position in eine ineinander eintauchende bzw. einander übergreifende Position, wobei auch die Öffnung 5 von einem entsprechenden Verstärkungsring definiert ist. Im in Fig. 2 dargestellten, zusammengefalteten Zustand liegt das zwischen den Verstärkungsringen 8 und 9 liegende Material in einem gefalteten Zustand in einer vordefinierten Lage, so daß durch eine Zugbeaufschlagung im Bereich der Öffnung 5 aus der in Fig. 2 dargestellten Transportposition wiederum die in Fig. 1 dargestellte Gebrauchsposition erzielbar ist.

Für den Fall einer Ausbildung des Faltenbalgs 2 mit unterschiedlichen Volumina kann eine entsprechend der Formgebung des Faltenbalgs 2 abgeänderte Anzahl von Verstärkungen bzw. -elementen 8 bzw. 9 vorgesehen sein.

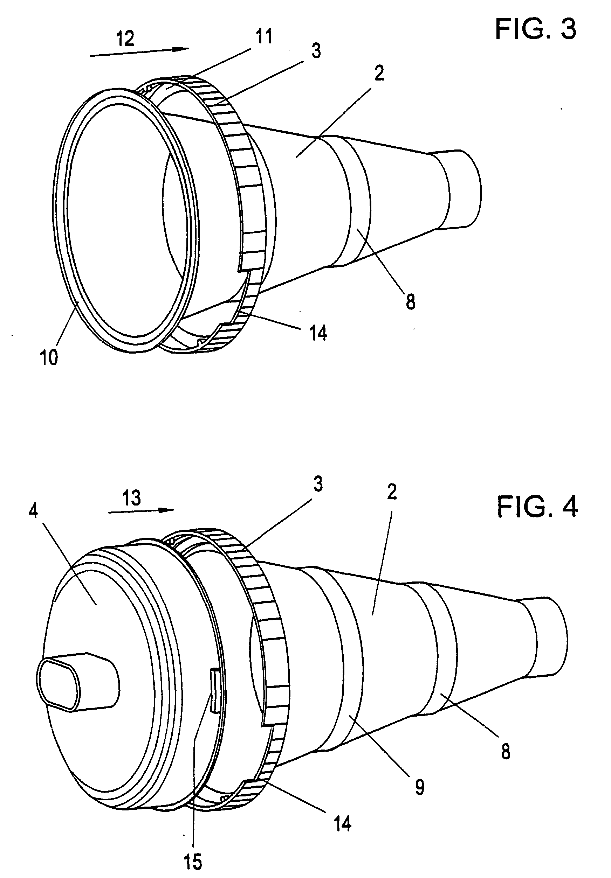

Bei den Explosionsdarstellungen gemäß den Fig. 3 und 4 ist ersichtlich, daß der Faltenbalg 2 an seinem den größeren Querschnitt aufweisenden Ende mit einem Wulst bzw. Flansch 10 versehen ist, welcher von einem entsprechenden Vorsprung bzw. Flansch 11 des Halterings 3 übergriffen wird, wobei in Fig. 3 bei einer Bewegung des Halterings 3 im Sinn des Pfeils 12 der Haltering vom Faltenbalg, insbesondere vom Wulst bzw. Flansch 10, gelöst wird.

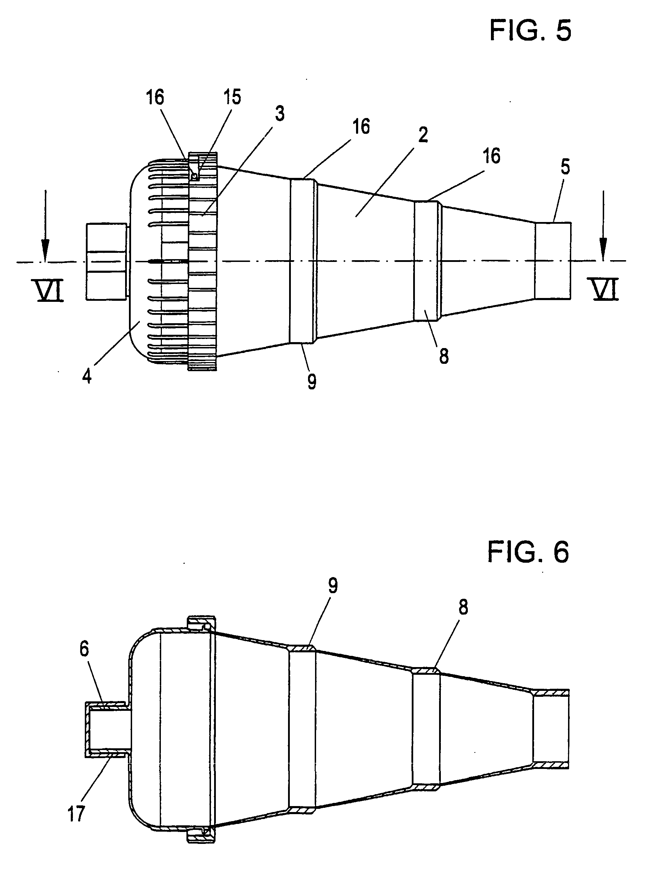

In Fig. 4 ist schematisch der Zusammenbauvorgang des Faltenbalgs 2 mit dem daran festgelegten Haltering 3 mit dem topfartigen Gehäuse 4 in Richtung des Pfeils 13 dargestellt. Die lösbare Verbindung zwischen dem Haltering 3 und dem Gehäuse 4 erfolgt beispielsweise über einen Schraubverschluß bzw. einen Bajonettverschluß, wobei zu diesem Zweck der Haltering mit entsprechenden Ausnehmungen 14 und das Gehäuse mit komplementären Profilen 15 versehen ist, welche in weiterer Folge und näher im Detail in den Fig. 5 und 6 ersichtlich sind.

In den schematischen Darstellungen gemäß den Fig. 5 und 6 ist erkennbar, daß die ringartigen Verstärkungen 8 bzw. 9 an der Außenfläche des Faltenbalgs 2 einen abgesetzten Bereich bzw. eine Stufe 16 ausbilden. Aus der Schnittdarstellung gemäß Fig. 6 ist weiters ersichtlich, daß die ringartigen Verstärkungen 8 und 9 einen gegenüber den unmittelbar anschließenden Bereichen des Faltenbalgs 2 vergrößerten Materialquerschnitt aufweisen, wobei für ein einfaches Falten von der in Fig. 1 dargestellten Gebrauchsposition zu der in Fig. 2 dargestellten Transportposition in dem an die ringartigen Verstärkungen 8 und 9 anschließenden Bereich der Faltenbalg 2 darüber hinaus mit einem entsprechend verjüngten Querschnitt ausgebildet sein kann, um den Faltvorgang zu erleichtern bzw. zu unterstützen.

Bei der Darstellung gemäß den Fig. 5 und 6 sind wiederum entsprechende Ausnehmungen 15 am Haltering 3 sowie Justier- bzw. Rastelemente 16 am Gehäuse 4 angedeutet, um den gewünschten Verriegelungsmechanismus bzw. Bajonettverschluß auszubilden.

Darüber hinaus ist in Fig. 5 und 6 eine zusätzliche Abdeckung 17 für die das Mundstück definierende Öffnung 6 angedeutet, wobei eine ähnliche Abdeckung auch an der Öffnung 5 vorgesehen sein kann, um ein Eindringen von Fremdmaterial beispielsweise in der Transportstellung zu vermeiden.

Durch die einfache Lösbarkeit des Gehäuses 4 vom Haltering 3 sowie des Halterings 3 vom Faltenbalg 2, wie dies in den Fig. 3 und 4 angedeutet ist, kann eine einfache und ordnungsgemäße Reinigung des Innenraums der durch den Faltenbalg 2 definierten Kammer des Inhalationshilfsgeräts 1 erfolgen, wobei auch ein entsprechend einfacher und zuverlässiger, dichtender Zusammenbau möglich ist.

## Patentansprüche

1. Inhalationshilfsgerät zur Verwendung in Verbindung mit einem Aerosolabgabegerät, umfassend eine Kammer, welche mit einer Öffnung (5) für die Verbindung mit einem Aerosolabgabegerät und mit einer Öffnung (6) mit einem Mundstück zum Abziehen des Aerosols ausgebildet ist, wobei die Kammer mit veränderbarem Volumen ausgebildet ist, wobei die Kammer von einem sich konisch verjüngenden Faltenbalg (2) aus einem durchgehenden, elastischen Material begrenzt ist, **dadurch gekennzeichnet, daß** der Faltenbalg (2) wenigstens eine ringartige Verstärkung (8, 9) mit gegenüber den anschließenden Bereichen des Faltenbalgs (2) verstärktem Materialquerschnitt aufweist.

2. Inhalationshilfsgerät nach Anspruch 1, **dadurch gekennzeichnet, daß** der Faltenbalg (2) aus einem Silikon hergestellt ist.

3. Inhalationshilfsgerät nach Anspruch 1 oder 2, **dadurch gekennzeichnet, daß** die Außenfläche des Faltenbalgs (2) im Bereich der zum sich verjüngenden Ende weisenden Stirnfläche (n) der ringartigen Verstärkung (en) (8, 9) stufenartig bzw. abgesetzt ausgebildet ist.

4. Inhalationshilfsgerät nach Anspruch 1, 2 oder 3, **dadurch gekennzeichnet, daß** der Faltenbalg (2) im an die Verstärkungen(en) (8, 9) unmittelbar anschließenden Bereich, insbesondere an der zum Inneren gewandten Seitenfläche der Kammer, mit verringertem Materialquerschnitt ausgebildet ist.

5. Inhalationshilfsgerät nach einem der Ansprüche 1 bis 4, **dadurch gekennzeichnet, daß** der Faltenbalg (2) in zusammengefaltetem Zustand in einem topfartigen, starren Gehäuse (4) aufnehmbar ist, welches das Mundstück (6) zum Abziehen des Aerosols aus der Kammer aufweist.

6. Inhalationshilfsgarät nach einem der Ansprüche 1 bis 5, **dadurch gekennzeichnet, daß** der Faltenbalg (2) an seinem einen größeren Querschnitt aufweisenden Ende mit einem Wulst bzw. Flansch (10) versehen ist, an welchem ein Haltering (3) festlegbar ist.

7. Inhalationshilfsgerät nach Anspruch 5 oder 6, **dadurch gekennzeichnet, daß** der Haltering (3) mit dem Gehäuse (4) zur Aufnahme des Faltenbalgs (2) insbesondere lösbar verbindbar ist.

8. Inhalationshilfsgerät nach Anspruch 7, **dadurch gekennzeichnet, daß** die Verbindung zwischen dem Haltering (3) und dem Gehäuse (4) von einem Schraubverschluß oder einem Bajonettverschluß gebildet ist.

9. Inhalationshilfsgerät nach einem der Ansprüche 5 bis 8, **dadurch gekennzeichnet, daß** am Haltering (3) und/oder dem Gehäuse (4) Markierungen bzw. Rast- oder Justierelemente (15, 16) vorgesehen sind.

10. Inhalationshilfsgerät nach einem der Ansprüche 1 bis 9, **dadurch gekennzeichnet, daß** Abdeck- bzw. Verschlußeinrichtungen (17) für die Öffnungen (5, 6) der Kammer vorgesehen sind.

## Claims

1. An inhalation aid for use in connection with an aerosol dispenser, comprising a chamber designed with an opening (5) for connection to an aerosol dispenser and an opening (6) including a mouthpiece for drawing the aerosol, said chamber being designed with a variable volume and delimited by a conically tapering expansion bellows (2) made of a continuous, elastic material, **characterized in that** the expansion bellows (2) comprises at least one ring-shaped reinforcement (8, 9) having a material cross section increased relative to the adjacent portions of the expansion bellows (2).

2. An inhalation aid according to claim 1, **characterized in that** the expansion bellows (2) is made of silicone.

3. An inhalation aid according to claim 1 or 2, **characterized in that** the outer surface of the expansion bellows (2) is designed to be stepped or offset in the region of the end face(s) of the ring-shaped reinforcement(s) (8, 9) oriented towards the tapering end.

4. An inhalation aid according to claim 1, 2 or 3, **characterized in that** the expansion bellows (2) is designed to have a reduced material cross section in its portion immediately following the reinforcement(s) (8, 9), particularly on the chamber side face oriented towards the interior.

5. An inhalation aid according to any one of claims 1 to 4, **characterized in that** the expansion bellows (2), in its folded state, is capable of being received in a pot-like, rigid housing (4) which comprises the mouthpiece (6) for drawing aerosol from the chamber.

6. An inhalation aid according to any one of claims 1 to 5, **characterized in that** the expansion bellows (2), on its end having a larger cross section, is provided with a bead or flange (10) to which a retaining ring (3) is fixable.

7. An inhalation aid according to claim 5 or 6, **characterized in that** the retaining ring (3) is connectable and, in particular, detachably connectable with the housing (4) provided to receive the expansion bellows (2).

8. An inhalation aid according to claim 7, **characterized in that** the connection between the retaining ring (3) and the housing (4) is comprised of a screw connection or bayonet catch.

9. An inhalation aid according to any one of claims 5 to 8, **characterized in that** markings, or snap-on or adjusting elements (15, 16), are provided on the retaining ring (3) and/or housing (4).

10. An inhalation aid according to any one of claims 1 to 9, **characterized in that** cover or closing means (17) are provided for the openings (5, 6) of the chamber.

## Revendications

1. Appareil auxiliaire d'inhalation pour utilisation en liaison avec un appareil de distribution par aérosol, comprenant une chambre, laquelle est réalisée avec une ouverture (5) pour la liaison à un appareil de distribution par aérosol et avec une ouverture (6) dotée d'un embout pour soutirer l'aérosol, la chambre étant conçue avec un volume variable, la chambre étant délimitée par un soufflet (2) se rétrécissant de façon conique en un matériau continu et élastique, **caractérisé en ce que** le soufflet (2) comprend au moins un renforcement annulaire (8, 9) doté d'une section de matériau renforcée par rapport aux zones contiguës du soufflet (2).

2. Appareil auxiliaire d'inhalation selon la revendication 1, **caractérisé en ce que** le soufflet (2) est fabriqué en silicone.

3. Appareil auxiliaire d'inhalation selon la revendication 1 ou 2, **caractérisé en ce que** la surface extérieure du soufflet (2) est réalisée de façon échelonnée ou étagée dans la zone de la (des) surface(s) frontale(s) du(des) renforcement(s) annulaire(s) (8, 9) orientée(s) vers l'extrémité se rétrécissant.

4. Appareil auxiliaire d'inhalation selon la revendication 1, 2, ou 3, **caractérisé en ce que** le soufflet (2) est réalisé avec une section de matériau réduite dans la zone directement contiguë au(x) renforcement(s) (8, 9), notamment sur la surface latérale de la chambre tournée vers l'intérieur.

5. Appareil auxiliaire d'inhalation selon l'une quelconque des revendications 1 à 4, **caractérisé en ce que** le soufflet (2), à l'état replié, peut être logé dans un boîtier rigide (4) du type pot, lequel comprend l'embouchure (6) destinée à soutirer l'aérosol de la chambre.

6. Appareil auxiliaire d'inhalation selon l'une quelconque des revendications 1 à 5, **caractérisé en ce que** le soufflet (2) est pourvu, sur une de ses extrémités comprenant une section transversale plus importante, d'un bourrelet ou d'un rebord (10), auquel peut être fixé un anneau de retenue (3).

7. Appareil auxiliaire d'inhalation selon la revendication 5 ou 6, **caractérisé en ce que** l'anneau de retenue (3) peut être relié notamment de manière amovible au boîtier (4) destiné à loger le soufflet (2).

8. Appareil auxiliaire d'inhalation selon la revendication 7, **caractérisé en ce que** la liaison entre l'anneau de retenue (3) et le boîtier (4) est formée par une fermeture à vis ou une fermeture à baïonnette.

9. Appareil auxiliaire d'inhalation selon l'une quelconque des revendications 5 à 8, **caractérisé en ce que** des marques ou des éléments d'enclenchement ou d'alignement (15, 16) sont prévus sur l'anneau de retenue (3) et/ou le boîtier (4).

10. Appareil auxiliaire d'inhalation selon l'une quelconque des revendications 1 à 9, **caractérisé en ce que** des systèmes de recouvrement ou de fermeture (17) sont prévus pour les ouvertures (5, 6) de la chambre.
